# EUROPEAN PATENT APPLICATION

(11) **EP 1 543 854 A1**
(43) Date of publication of application: **22.06.2005**
(21) Application number: 03388087.3
(22) Date of filing: 16.12.2003
(51) Int. Cl.: A61M 5/158

(54) **Vibrating injection needle and method for detecting the presence of medicament therein**

(71) Applicant: NOVO NORDISK A/S, 2880 Bagsvaerd (DK)
(72) Inventor: Glejbol, Kristian, 2600 Glostrup (DK); Smedegaard, Jorgen K., 2720 Vanlose (DK); Pedersen, Thomas Orts, 2800 Lyngby (DK); Poulsen, Jens Ulrik, 2830 Virum (DK); Kallesoe, Bjarne, Skovmose, 2800 Bagsvaerd (DK); Thomsen, Jon Juel Technical University of Denmark, 2800 Lyngby (DK)

(57) **Abstract**

The invention relates to a delivery device and a method which has the ability to automatically decide when an air shot has been performed. The method is based on the principle of monitoring the change in an initial resonance frequency of an injection needle during the filling of the injection needle. When the change of the initial resonance frequency becomes as expected, the injection needle is considered filled with liquid and the air shot is fulfilled. The expected change can be that of reaching a predetermined value or that of reaching a stable and constant level of values indicating that the resonance frequency of the filled injection needle is reached. Depending on the criteria for the expected change, a system able to identify if the needle is partly filled or if the needle is 100 % filled can be set up.

## Description

### THE TECHNICAL FIELD OF THE INVENTION:

The invention relates to a delivery device for administration of a therapeutic liquid to a living body and more particularly to a delivery device having a hollow conduit such as an injection needle which is vibrated prior to injection.

The invention further relates to a method which can identify the location of a hollow conduit and a method which can automatically identify whether liquid is present in the hollow conduit.

### DESCRIPTION OF RELATED ART:

To carry out injections of therapeutic liquids into living tissue different delivery devices are employed. The simplest form of delivery device is the well known hypodermic syringe. However more sophisticated delivery devices including various electronic features find widespread use in particular for injecting insulin in self treatment of diabetes.

Prior to injecting a liquid medicament into a living body the medical delivery device needs to be emptied for air in order to enhance comfort for the user and to increase the precision of the delivered dose.

Air is often captured in the interior of the medical delivery device and especially in the path the therapeutic liquid have to travel during injection. Air is usually sucked into the medical delivery device either when filling the medical delivery device or if the medical delivery device is left with the injection needle mounted on the device e.g. due to temperature variations. It is therefore recommendable to ensure that both the injection needle and the medical delivery device are emptied for trapped air before a dose is set and injected.

To get rid of possible trapped air a small dose of medicine is set and an injection operation is made without insertion of the needle into the tissue. By this so called "air shot", air is driven out through the needle. At the point of the needle it can easily be seen if all air has been expelled. If a fine liquid jet is not seen at the end of the air shot a new small dose must be set and a new air shot be made until such a jet is seen.

To avoid having to perform repetitive settings of a small dose and subsequent air shot, the user may feel an impulse to set a somewhat larger dose. This often results in an unnecessary waste of expensive medicament and has lead to the development of medical delivery devices having a separate air shot button by which a predetermined amount can be expelled.

Such a medical device is disclosed in WO 02/058766. This medical delivery device comprises an electrical motor which is activated by a separate prime button to expel a predetermined small dose of liquid medicament. Albeit being provided with a separate air shot button, this delivery device is not fully automated and the user still have to determine how many times the motor should be activated.

Further a medical delivery device comprising a needle insertion sensor is disclosed in WO 03/092487. By this sensor it is possible to determine if the injection needle is completely inserted into the tissue of a living body. Since a part of the sensor is located on the delivery device itself it is only possible to determine if the injection needle is fully inserted i.e. the delivery device is in contact with the surface of the tissue.

A traditional hypodermic syringe with a vibrator is disclosed in JP 09-239031. The vibrator is mounted such that the injection needle vibrates during injection. According to the disclosure such ultrasonic vibration of the injection needle during injection reduces the pain felt when inserting the injection needle into the human body.

### DESCRIPTION OF THE INVENTION:

It is an object of the present invention to provide a medical delivery device which can differentiate between being inserted into living tissue or not, even if the injection needle of the delivery device is only partly inserted.

It is a further object to provide a medical delivery device which has the ability to automatically decide when an air shot has been performed.

### Description of Claim 1:

By inducing vibrations in the hollow conduit before injection and identify an initial resonance frequency of the hollow conduit when it is free in the air, it will be possible to identify a dampening of the vibration by identifying a change in the initial resonance frequency.

A change of the initial resonance frequency will occur when the mass of the vibrating conduit is changed or if the conduit comes into contact with an object dampening the vibrations.

An initial resonance frequency identified when the hollow conduit is in the free air would decrease significantly when the hollow conduit is inserted into living tissue. The difference in the frequency will reflect the mechanical properties of the living tissue and help identifying the media in which the hollow conduit is inserted, or at least separate between two significant different mediums such as air and living tissue.

### Description of Claim 2:

Prior to starting the priming, the hollow conduit is vibrated and the initial resonance frequency is identified. This initial resonance frequency is associated with an empty needle and identifies a first stage of operation. The initial resonance frequency identified is preferably but not necessarily the first mode resonance frequency.

The identification of the initial resonance frequency defines the first stage of operation, at which stage the filling of the hollow conduit is initiated. The hollow conduit is slowly filled with liquid medicament from the cartridge by moving the piston forward inside the cartridge thereby changing the mass of the hollow conduit. This forward movement is in a preferred embodiment executed by an electrical motor but can off cause be done by other means. The forward movement could even be done manually and the priming mechanism could in this case release a signal e.g. visual or audio, to the user when the priming is done.

During the filling of the needle, the frequency of the vibrating hollow conduit is continuously monitored, and when a second frequency associated with a filled needle is identified the filling of the hollow conduit is halted at what is identified as a second stage of operation.

The boundary or transition between the first stage of operation where the hollow conduit is empty and the second stage of operation where the hollow conduit is filled is defined by the manufacture of the delivery device and need not be defined such that the hollow conduit is 100 % filled before entering the second stage, a lower degree of filled conduit could be acceptable.

The priming is based on the principle of monitoring the change in the initial resonance frequency of a hollow conduit during the filling of the hollow conduit. When the change of the initial resonance frequency becomes as expected, the hollow conduit is considered filled with liquid and the priming is fulfilled. The expected change can however be expressed in a number of different ways as in the following claims.

### Description of Claim 3:

The boarder line or transition between the first stage of operation and the second stage of operation is according to an embodiment of the invention defined by the difference between the initial resonance frequency and the second frequency reaching a predetermined value.

This predetermined value can either be programmed in a micro processor present in the delivery device as a numerical value or it can be programmed as an algorithm such that the value can be calculated by the micro processor. It could e.g. be a percentage of the initial resonance frequency such that the continuous delivery into the interior of the conduit would be halted when the second frequency is a predetermined percentage different from the initial frequency.

### Description of Claim 4:

If the second frequency is the resonance frequency of a filled hollow conduit, a stable level of frequencies easy to measure can be obtained.

The resonance frequency of the filled hollow conduit is preferably the first mode resonance frequency, but could off cause be any mode of resonance frequency. It is further preferred that the initial resonance frequency and the resonance frequency of the filled conduit i.e. the second frequency is the same mode of resonance frequency

### Description of Claim 5:

When the second resonance frequency has been stable at a constant level for a period of time, the second stage of operation is identified and the priming operation i.e. the continuous delivery is halted.

### Description of Claim 6:

A plurality of different transducers can be employed for excitation of the hollow conduit. The vibrator could e.g. be based on an electromechanical principle. Since a sensor for sensing the frequency of the vibrated needle is also needed a preferred solution would be to use a transmitter such as a piezo electric material. A piezo electric material can be utilized both as a transmitter for vibrating the needle and as a sensor or receiver for sensing the vibrations. The preferred range is 1 to 30 KHz.

### Description of Claim 7:

In a preferred embodiment an electrical motor is used for the continuously delivery of the liquid medicament from the cartridge through the hollow conduit. When using an electric motor the control can be electronic and therefore coupled to the sensor monitoring the vibrations in a way such that the priming can be fully automated.

If no electrical motor were present, the user would have to prime the delivery device manually and to be informed when the second stage of operation were reached.

### Description of Claim 8-13:

Two methods sharing the same inventive idea are described.

The first method comprises the steps of:
(i) exciting the hollow conduit
(ii) identifying the initial resonance frequency of the vibrated hollow conduit
(iii) identifying a change in the initial resonance frequency.

By exciting or vibrating the hollow conduit and monitor the change in the initial resonance frequency it is possible to determine if the hollow conduit have moved from being free to vibrate in the air to be inserted into a media which dampens the frequency. If an injection needle is referred to, the media could be the living tissue of a human. In this case it would be possible to identify if the injection needle is inside or outside the living tissue. This identification will be possible the same instant the injection needle touches the surface of the tissue.

A system were it would be possible to identify the type of tissue the injection needle is inserted into, and even high deep into the living tissue the injection needle is inserted could be based on this principle.

The second method referred to comprises the steps of:
(i) identifying an initial resonance frequency associated with an empty hollow conduit at a first step of operation,
(ii) continuously delivering a liquid medicament into the conduit,
(iii) identifying a second frequency associated with a filled hollow conduit at a second stage of operation,
(iv) halting the continuous delivery of liquid medicament into the hollow conduit when the second stage is identified.

Once the initial resonance frequency has been identified, the filling of the hollow conduit can commence. During the filling the change in frequency is monitored such that the continuous delivery of liquid medicament into the hollow conduit can be halted once the change in the frequency is such that the criteria for entering into the second stage of operation are fulfilled.

In an embodiment of the method the criteria is that the difference between the initial resonance frequency and the second frequency reaches a predetermined value, which value can be decided either numerical or by an algorithm. The value can be set such that the hollow conduit not necessarily has to be 100 % filled when the predetermined value is reached.

In a further embodiment of the method the criteria is that the second frequency must reach a stable and constant level indicating that it has reached a new, stable resonance frequency whereby the hollow conduit is 100 % filled.

By vibrating the hollow conduit during injection, it will in addition be possible to eliminate the pain perception usually connected with needle injections.

All though the invention is explained in connection with an injection needle, the term hollow conduit is meant to include other kinds of conduits. An inserter for inserting a blood glucose sensor into living tissue could also be vibrated prior to and/or during insertion into the tissue in order to determine if the inserter is inserted or not. The pain lowering aspect of the invention could also be used for such or other applications.

The ability to identify whether liquid is present in the hollow conduit or not can be integrated with the control of a sophisticated electronic delivery device in order to enhance the overall safety and introduce even more intelligent systems. If no liquid is present in the hollow conduit for a period of time, it could indicate a faulty situation. This could e.g. be that the cartridge is empty, the pathway for the liquid is blocked or a mechanical fault preventing the forward movement of the piston rod has occurred.

### BRIEF DESCRIPTION OF THE DRAWINGS:

The invention will be explained more fully below in connection with a preferred embodiment and with reference to the drawings in which:
Figure 1 shows a schematic view of a delivery device according to the invention.
Figure 2 shows a schematic view of the injection needle connected to the delivery device according to the invention.
Figure 3 shows the injection needle subjected to transversal vibrations.
Figure 4 shows a graphical view of the relation between the effect and the frequency.
Figure 5 shows a graphical view of the frequency over a period of time.
Figure 6 shows the injection needle inserted into living tissue.

The figures are schematic and simplified for clarity, and they just show details, which are essential to the understanding of the invention, while other details are left out. Throughout, the same reference numerals are used for identical or corresponding parts.

### DETAILED DESCRIPTION OF EMBODIMENT:

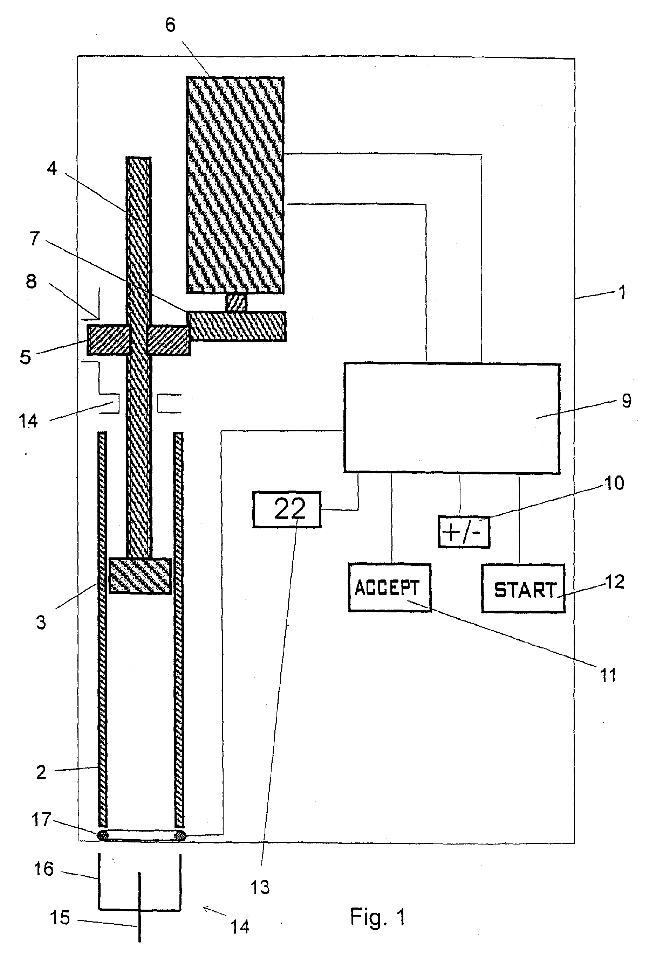

Figure 1 discloses a delivery device comprising a housing 1 supporting a cartridge 2 containing a liquid medicament. The delivery device further comprises an electrical motor 6 which through a gearing 5, 7 is able to move a piston rod 4 forward.

In the disclosed embodiment this forward movement is caused by rotating the nut member 5 having an interior thread mating the exterior thread on the piston rod 4. By preventing the nut member 5 from longitudinal movement within the housing 1 e.g. by locking the nut member 5 to the housing 5 through an aperture 8 in the housing 1, and at the same time rotational locking the piston rod 4 to the housing 1, e.g. by having a protrusion 14 in the housing 1 interfacing a non-circular shape on the piston rod 4, it will be possible to screw the piston rod 4 forward by rotating the nut member 5.

The nut member 5 is rotated by the gearing wheel 7 which is connected to the axis of the electrical motor 6. The operation of the motor 6 is controlled by the microprocessor 9 which receives various inputs from the user through a number of user interfaces 10, 11, 12, 13. The automatic priming function described herein is also executed by the microprocessor 9.

The user interfaces 10, 11, 12, 13 could in a preferred embodiment be a dose setting button 10 on which the dose size can be dialled up or down, an accept button 11 to accept the set dose and to arm the system prior to injecting and an injection button 12 used to inject the set dose after the delivery device has been set and the dose size has been confirmed. Further a display 13 displaying the set dose and other features are available.

When the piston rod 4 is screwed forward it moves a rubber piston 3 inside the cartridge 2 thereby expelling an amount of the liquid medicament contained in the cartridge 2 out through an injection needle 14 connected to the housing 1 of the delivery device.

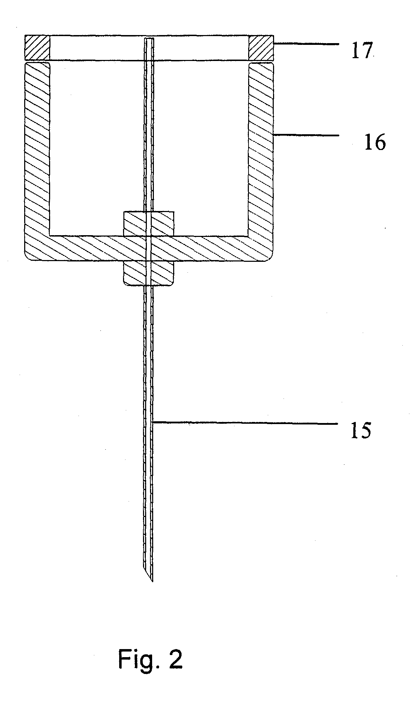

Such injection needles 14 shown in greater details in figure 2 usually comprises a metallic hollow cannula 15 firmly mounted in a hub 16 moulded from a polymeric material.

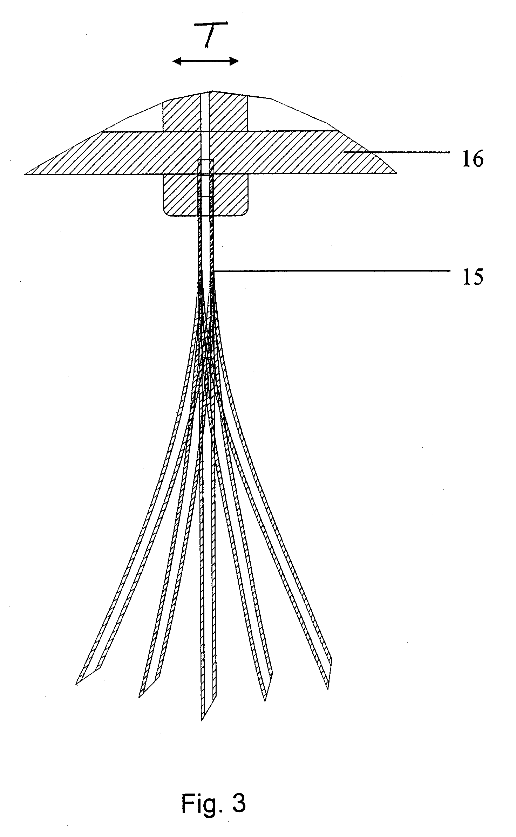

At the interface between the injection needle 14 and the housing 1 of the delivery device a vibrator such as a piezo electric material 17 is arranged. The piezo electric material 17 could e.g. be a crystal, a ceramic material or a polymer such as PVDF (Polyvinylidenediflouride). When driving the piezo material 17 the injection needle 14 will be vibrated as shown in figure 3. At the same time the piezo electric material 17 can be used as a sensor measuring the response from the injection needle 14.

For priming the system the injection needle 14 is vibrated by applying electric power to the piezo material 17. By sweeping the piezo material 17 from a low value of frequencies to a higher value of frequencies a change in the power occurs when the injection needle 14 vibrates at its resonance frequency. When the injection needle 14 and the piezo electric material 17 is rigidly connected the resonance occurring when the injection needle 14 vibrates at its resonance frequency will contribute to the overall power consumption of the rigid system whereby the power needed to the excitation of the piezo electric material will decrease. If e.g. an AC current at a constant amplitude is used, the power consumption will decrease when the resonance frequency is identified.

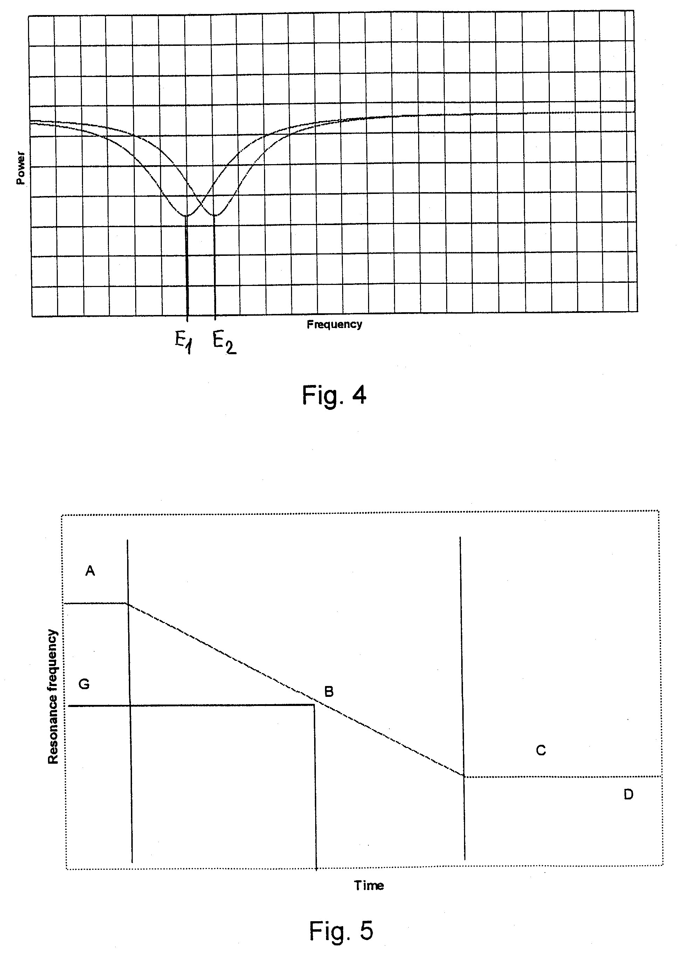

As disclosed in figure 4, the power depictured on the Y-axis is kept constant while the range of frequencies plotted on the X-axis is swept preferably from a low value to a high value. When the vibrating injection needle 14 reaches its resonance frequency, the power Y absorbed by the piezo material 17 will fall considerable as indicated at E1.

Experiments indicate that the first mode resonance frequency is the preferred to use. Measuring the resonance frequency for first mode transverse vibrations of a number of NovoFine® injection needles from Novo Nordisk A/S has been identified.

| | | | |
|---|---|---|---|
| NovoFine® Needle | 31 G, 6 mm | 30 G , 6 mm | 30 G , 8 mm |
| Resonance frequency, empty needle | 5447 Hz | 5880 Hz | 3921 Hz |

Once the micro-processor 9 has determined the initial resonance frequency of the empty injection needle 14, the electric motor 6 will be activated slowly forcing the piston rod 4 and the rubber piston 3 forward. This forward movement will force the air trapped in the cartridge 2 out through the injection needle 14. Once all the air has escaped, the injection needle 14 will be slowly filled with the liquid medicament in the cartridge 2.

The filled injection needle 14 has a different resonance frequency from an empty injection needle 14. The resonance frequency of a filled injection needle 14 is always lower than the frequency of an empty injection needle due to the increased mass of the injection needle 14. The difference in the resonance frequency depends on the type of vibration the injection needle 14 is submitted to. In mathematical modelling the difference in resonance frequency for an injection needle is calculated to be approximately 2 % for transversal and longitudinal vibrations whereas it is less than 1 % for torsional vibrations. Since transversal vibrations at the same time are the easiest kind to measure, transversal vibrations or at least vibrations predominantly comprising a transverse component are to be preferred for a priming system as indicated at T in figure 3.

When subjecting the above injection needles to transversal vibrations once the injection needles are filled, the following picture emerges

| | | | |
|---|---|---|---|
| NovoFine® Needle | 31 G , 6 mm | 30 G , 6 mm | 30 G , 8 mm |
| Resonance frequency, empty needle (A) | 5447 Hz | 5880 Hz | 3921 Hz |
| Resonance frequency, full needle (C) | 5365 Hz | 5706 Hz | 3816 Hz |
| Difference in % | 1,51 % | 2,96 % | 2,67 % |

The difference between the resonance frequency of an empty injection needle and a full injection needle is large enough to be identified. By constantly monitoring the change in frequency by sweeping the range of frequencies, the resonance frequency of the filled injection needle 14 can be identified by a second fall in the effect absorbed by the piezo material 17. This fall is indicated as E2 in figure 4.

The priming system is further explained in relation to figure 5 where the graph D shows the time on the X-axis and the frequency on the Y-axis.

First the resonance frequency of an empty injection needle 14 is identified, represented by the straight curve A. Hereafter the injection needle 14 is slowly filled with the liquid medicament which will cause the natural frequency to alter, represented by the sloping curve B. Once the injection needle 14 has been filled a new straight curve C will emerge since the filled injection needle 14 has a different resonance frequency which will appear when the injection needle is 100 % full. There are two stable conditions, empty injection needle and filled injection needle. When filling the injection needle 14 the frequency will alter because each degree of filling has it own resonance frequency. However since an injection needle 14 can not be more than 100 % filled, a new stable condition will emerge once the injection needle 14 is filled.

Once this new constant and stable resonance frequency has been identified the electric motor is halted and the priming is completed.

In an alternative system the guidance to halt the motor could be a predetermined fall in the frequency. First the resonance frequency of an empty injection needle could be identified upon which the filling of the injection needle could be initiated. When constantly sweeping the frequency, the motor 6 could be halted when a predetermined fall in the frequency has been identified as illustrated by G in figure 4.

The system described could in addition be used for employing more intelligent systems. If e.g. the electric motor 6 is up and running but the piezo electric material 17 detects that the hollow conduit 14 is continuing to stay at its initial resonance frequency this would indicate a fault on the system such as no therapeutic liquid being delivered to the hollow conduit 14.

In an embodiment of the invention excitation of the hollow conduit 14 need not to be a time-harmonic sine sweep. The piezo electric material 17 could be excited by a short impulse where after the piezo is switched to sensor mode and the resonance frequency is identified from the free decoy response. In this way both the resonance frequency of the empty injection needle and the resonance frequency of the filled injection needle can be identified.

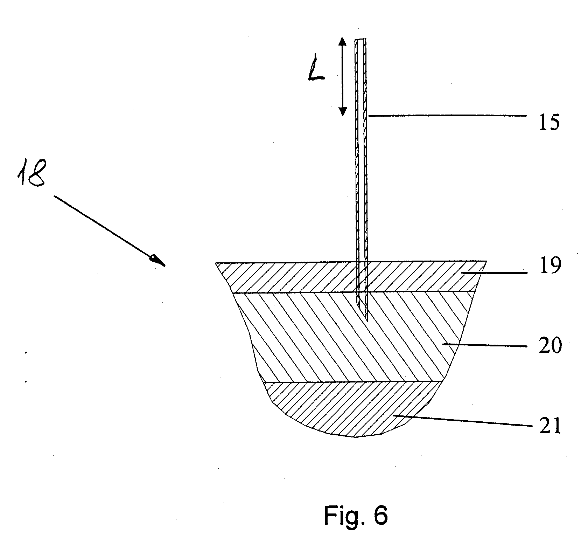

Figure 6 discloses the injection needle 14 with the needle cannula 15 inserted into the tissue 18 of a living body. Such tissue comprises of the epidermis 19, the dermis 20 and the subcutis 21. Liquids medicaments such as insulin are usually injected into the subcutis layer 21.

It is believed that the pain associated with a needle insertion can be reduced if the friction between the injection needle 14 and the tissue 18 can be reduced. For this purpose injection needles 14 are usually coated with a silicone based substance.

When injecting into the tissue 18 of a living body a rather high force is needed to force the injection needle 14 through the epidermis 19 due to the penetration resistance of the epidermis 19. Once the epidermis19 is penetrated a substantially lower force is needed to continue driving the injection needle 14 further into the dermis 20 and the subcutis layer 21. This indicates that the insertion force needed for injecting a substance such as insulin into the subcutis layer 14 comprises of many components.

For the purpose of lowering the friction between the injection needle 14 and the surrounding tissue 18 which will lead to a lowering of the insertion force, high frequency longitudinal vibrations could be superposed to the injection needle 14 during insertion into the living tissue 18. In soft tissue 18 the character of the friction occurring between the injection needle 14 and the tissue 18 changes when the injection needle 14 is vibrated at a high frequency as opposed to being inserted at a constant, non-vibrating velocity. High frequency vibrations of the injection needle 14 will change the dry friction into viscous dampening which is believed to reduce the pain felt during injection. This phenomenon is known from DD 22029 which describes a solid steel wire being introduced to a living body using ultrasonic vibrations.

Using high frequency vibrations generated by a piezo electric material 17 permanently secured in the housing 1 of an injection device provides a solution that would probably be very beneficial for lowering the injection pain associated with pen systems used for self treatment of diabetes.

Some preferred embodiments have been shown in the foregoing, but it should be stressed that the invention is not limited to these, but may be embodied in other ways within the subject matter defined in the following claims. All though different modes of vibrations are specified for certain applications, the claims are not limited to any specific mode of vibration.

## Claims

1. A delivery device for delivering a liquid medicament, comprising:
- a housing (1) for supporting a cartridge (2) containing the liquid medicament to be delivered,
- a hollow conduit such as an injection needle (14) adapted to communicate with the interior of the cartridge (2), and to be filled with the liquid medicament from the cartridge (2),
**Characterized in** further comprising:
- a transducer (17) for inducing vibrations the hollow conduit (14),
- a sensor (17) for sensing the vibrations induced in the hollow conduit (14),
- means (9) for identifying an initial resonance frequency of the vibrated hollow conduit (14), and
- means (9) for identifying a change in the initial resonance frequency

2. A delivery device for delivering a liquid medicament according to claim 1, **characterized in that**, the initial resonance frequency associated with an empty hollow conduit (14) is identified at a first stage of operation wherein the hollow conduit (14) is empty and a second frequency associated with a filled hollow conduit (14) is identified at a second stage of operation wherein the hollow conduit (14) is at least partly filed with the liquid medicament, and wherein the liquid medicament is continuously delivered into the interior of the hollow conduit (14) initiated at the first stage of operation and the continuous delivery is halted when the delivery device enters into the second stage of operation.

3. A delivery device for delivering a liquid medicament according to claim 2, **characterized in that**, the boundary or transition between the first stage of operation and the second stage of operation is defined by the difference between the initial resonance frequency and the second frequency, such that the continuous delivery is halted when the difference reaches a predetermined value.

4. A delivery device for delivering a liquid medicament according to claim 2, **characterized in that**, the second frequency is the resonance frequency of a filled injection needle (14).

5. A delivery device for delivering a liquid medicament according to claim 4, **characterized in that**, the boundary or transition between the first stage of operation and the second stage of operation is defined by the second frequency reaching a constant value for a predetermined period of time, such that the continuous delivery is halted when the second frequency has been constant for a period of time.

6. A delivery device for delivering a liquid medicament according to anyone of the preceding claims, **characterized in that**, the transducer (17) and the sensor is a piezo electric material provided in the housing (1).

7. A delivery device for delivering a liquid medicament according to anyone of the preceding claims, **characterized in that**, an electrical motor (6) is used for the continuously delivery of liquid medicament in to the conduit.

8. A method of identifying the location of a hollow conduit by vibrating the conduit, comprising the steps of:
(i) exciting the hollow conduit, and
(ii) identifying the initial resonance frequency of the vibrated hollow conduit
(iii) identifying a change in the initial resonance frequency.

9. A method of automatically identifying whether liquid is present in a hollow conduit by vibrating the conduit, comprising the steps of:
(i) identifying a initial resonance frequency associated with an empty hollow conduit (14) at a first stage of operation,
(ii) continuously delivering a liquid medicament into the conduit (14),
(iii) identifying a second frequency associated with a filled hollow conduit (14) at a second stage of operation,
(iiii) halting the continuous delivery of liquid medicament into the hollow conduit (14) when the second stage of operation is identified.

10. A method of automatically identifying whether liquid is present in a conduit according to claim 9, **characterized in that**, the boundary or transition between the first stage of operation and the second stage of operation is defined by the difference between the initial resonance frequency and the second frequency, such that the continues delivery is halted when the difference reaches a predetermined value.

11. A method of automatically identifying whether liquid is present in a conduit according to claim 9, **characterized in that**, the second frequency is the resonance frequency of a filled conduit (14).

12. A method of automatically identifying whether liquid is present in a conduit according to claim 11, **characterized in that**, the boundary or transition between the first stage of operation and the second stage of operation is defined by the second frequency reaching a constant value for a predetermined period of time, such that the continuous delivery is halted when the second frequency has been constant for a predetermined period of time.

13. A method of automatically identifying whether liquid is present in a conduit according to any one of the claims 8-12, **characterized in that**, the method further comprises the step of continuing vibrating the conduit (14) during insertion into the living tissue in order to reduce the pain perception of the living to be injected.
